# EUROPEAN PATENT APPLICATION

(11) **EP 4 431 024 A1**
(43) Date of publication of application: **18.09.2024**
(21) Application number: 23164439.4
(22) Date of filing: 27.03.2023
(51) Int. Cl.: A61B 8/00

(54) **3D ULTRASOUND IMAGING**

(30) Priority: 15.03.2023 US 202363452246 P
(71) Applicant: Koninklijke Philips N.V., 5656 AG Eindhoven (NL)
(72) Inventor: BARINK, Marco, 5656 AG Eindhoven (NL); LAVEZZO, Valentina, 5656 AG Eindhoven (NL); ROBERT, Jean-Luc Francois-Marie, 5656 AG Eindhoven (NL); ROESSL, Ewald, 5656 AG Eindhoven (NL)
(74) Representative: Philips Intellectual Property & Standards

(57) **Abstract**

A computer-implemented method of ultrasound imaging. The method comprises obtaining 3D volume acquisitions of an anatomical structure with a 3D ultrasound probe and, between 3D volume acquisitions, switching the 3D ultrasound probe to a 2D scan mode and controlling the 3D ultrasound probe, in the 2D scan mode, to obtain a 2D slice of the anatomical structure. The method further comprises extracting features from the 2D slice and generating a representation of an enhanced 3D volume, wherein the enhanced 3D volume combines a 3D volume acquisition and the features extracted from the 2D slice.

## Description

### FIELD OF THE INVENTION

The invention relates to the field of ultrasound imaging. In particular, the invention relates to the field of three-dimensional (3D) ultrasound imaging.

### BACKGROUND OF THE INVENTION

Two-dimensional (2D) ultrasound imaging can only show a slice of an anatomical structure. A clinician then has to determine/interpret how that slice is oriented with respect to the real 3D geometry of the anatomical structure. Misinterpretation of the visible structures in 2D, which can easily occur in the case of anatomic disorders, can lead to serious medical errors.

As such, three-dimensional ultrasound over time (3D+t ultrasound) has been used to provide the user an overview of a 3D anatomical structure in real time (e.g., a beating heart).

In the case of imaging the human heart, accurate imaging of the valve is required. A good overview of the surrounding structures, like the atrium and the ventricle, may also be required. However, a 3D overview of the heart, including surrounding structures, limits the resolution of the scan, specifically around a valve. As such, although the 3D ultrasound can provide a good overview of the surrounding structure, it may not show an accurate image of the valve.

Currently, the best type of 3D+t ultrasound scan is not able to visualize the leaflets tips sufficiently, leading to an interpretation of the leaflet length which is too low. The papillary muscles and the papillary tips are also not visible.

With ultrasound and other scan modalities, gating is often used to create a stable image. Averaging over multiple heartbeats can also lead to improved image quality. However, ultrasound probes move continuously (albeit slightly) during the heartbeat and especially in case of patients with structural heart disease, heartbeats do not have the same length and shape, which is a prerequisite for a good result with gating.

Thus, there is a need for an ultrasound imaging solution which can provide accurate imaging of an anatomical structure whilst also providing an overview of the surrounding structures.

### SUMMARY OF THE INVENTION

The invention is defined by the claims.

According to examples in accordance with an aspect of the invention, there is provided a computer-implemented method of ultrasound imaging, the method comprising:
controlling a 3D ultrasound probe, in a 3D scan mode, to obtain 3D volume acquisitions of an anatomical structure;
switching the 3D ultrasound probe to a 2D scan mode between 3D volume acquisitions and controlling the 3D ultrasound probe, in the 2D scan mode, to obtain a 2D slice of the anatomical structure;
extracting features from the 2D slice; and
generating a representation of an enhanced 3D volume, wherein the enhanced 3D volume combines a 3D volume acquisition and the features extracted from the 2D slice.

The probe is switched from the 3D scan mode to the 2D scan between sequential 3D volume acquisitions.

Ultrasound-based 3D volumes are typically obtained at a relatively low frame rate (e.g., around 15 frames per second) and with a relatively low spatial resolution. However, the 3D volumes are useful to obtain a holistic view of the anatomical structure.

In contrast, 2D ultrasound slices typically have a better spatial resolution (and thus improved geometric details) but only show a slice of the anatomical structure.

As such, it is proposed to combine both modalities and combine a 3D volume acquisition with features extracted from the 2D slice thereby to provide the improved geometric details from the 2D slice. This is achieved by switching between a 3D scan mode and a 2D scan mode on the same probe within a short time period (i.e., between 3D acquisitions).

The time between obtaining a 3D volume acquisition and obtaining a 2D slice may be, for example, less than 0.2 seconds, 0.1 seconds, 0.05 seconds or 0.02 seconds. For example, the 2D slice may be obtained between 0.2 seconds and 0.02 seconds from obtaining a 3D volume acquisition. The particular time may depend on user preferences, the 3D scan mode acquisition rate, the 2D scan mode acquisition rate and/or the processing resources available. The time between obtaining a 3D volume acquisition and obtaining a 2D slice may be a fraction of the time between 3D volume acquisitions (e.g., the fraction may between 1/5 and 1/20, such as 1/15).

Obtaining the 2D slice between 3D volume acquisitions, using the same probe, enables the 2D slice to be automatically registered to the 3D volume acquisitions as it can be assumed that they are taken from the same viewpoint. Thus, there is no need to register the 2D slice to the 3D volume acquisitions to combine the 3D volume acquisition with features from the 2D slice.

It has been realized that this is feasible because obtaining the 2D slices (e.g., between obtaining 3D volume acquisitions) does not add a significant amount of time to the overall workload. As such, the 2D slices can be obtained without significantly reducing the acquisition rate of the 3D volume.

The method may comprise continuously switching between controlling the 3D ultrasound probe in the 3D scan mode and the 2D scan mode.

The 2D ultrasound probe may be controlled to obtain a plurality of 2D slices at different target planes (e.g., different orientations) between 3D volume acquisitions.

The method may also comprise displaying the 2D slice. The 2D slice may be displayed together with the enhanced 3D volume.

The 3D volume acquisition used to generate the representation of the enhanced 3D volume may be the 3D volume acquisition obtained immediately before obtaining the 2D slice or the 3D volume acquisition obtained immediately after obtaining the 2D slice.

The representation of the enhanced 3D volume may comprise part, or all of, the geometry of the 3D volume and part, or all of, the geometry of the extracted features.

The method may comprise switching the 3D ultrasound probe to the 2D scan mode and controlling the 3D ultrasound probe, in the 2D scan mode, to obtain a 2D slice of the anatomical structure within 0.2 seconds from obtaining a 3D volume acquisition.

The method may further comprise controlling the 3D ultrasound probe, in the 2D scan mode, to obtain a second 2D slice at a different plane from the 2D slice based on the extracted features.

If the features in the 2D slice indicate that the 2D slice is not at a target plane, the probe can be controlled to obtain a second 2D slice at a different plane (e.g., a different orientation or different angle) to attempt to obtain a 2D slice closer to the target plane.

Relative positions/orientations between features can be used to estimate the plane of the 2D slice.

The method may further comprise switching the 3D ultrasound probe from the 2D scan mode to the 3D scan mode based on the extracted features.

The method may further comprise extracting volume features from a 3D volume acquisition, wherein switching the 3D ultrasound probe from the 3D scan mode to the 2D scan mode is based on the extracted volume features.

Relative positions/orientations between the volume features can be used to estimate the position of a target plane. The 3D probe could thus be controlled to obtain the 2D slice at the target plane.

The method may further comprise determining a target plane and corresponding acquisition parameters based on the extracted volume features, wherein the 3D ultrasound probe is controlled, in the 2D scan mode, to obtain a 2D slice of the anatomical structure at the target plane using the acquisition parameters.

Generating a representation of an enhanced 3D volume may comprise adapting the 3D volume acquisition to a first transparency and superimposing the 2D slice on the 3D volume acquisition.

The enhanced 3D volume can be displayed to a user. Thus, the full 3D volume of the anatomical structure can be visualized by the user whilst the higher resolution/accuracy features are also visible to the user. The superimposed 2D slice has a lower transparency than the 3D volume (or no transparency).

The method may further comprise switching the 3D ultrasound probe to a second 3D scan mode, after obtaining the 2D slice, and controlling the 3D ultrasound probe, in the second 3D scan mode, to obtain a second 3D volume acquisition of the anatomical structure, wherein the second 3D scan mode may have a higher beam density near the position of the plane of the 2D slice and a lower beam density away from the position of the plane of the 2D slice.

This provides more detail in the second 3D volume near the position of the 2D slice.

Alternatively, or additionally, the second 3D scan mode may have a higher beam density than the 3D scan mode at the position of a plane of the 2D slice.

The second 3D scan mode may have a higher pulse repetition interval than the (first) 3D scan mode.

This provides a higher frame rate for the second, 3D volume.

A processor may be configured to perform any step of the afore-mentioned methods.

The invention also provides a computer program carrier comprising computer program code means which, when executed on a processing system, cause the processing system to perform all of the steps of the method according to any of the preceding embodiments.

A computer program carrier may be, for example, a long-term storage product (e.g., a hard drive or a solid-state drive) or a short-term stream of data (e.g., a bitstream).

The invention also provides a system for ultrasound imaging, the system comprising a processor configured to:
control a 3D ultrasound probe, in a 3D scan mode, to obtain 3D volume acquisitions of an anatomical structure;
switch the 3D ultrasound probe to a 2D scan mode between 3D volume acquisitions and control the 3D ultrasound probe, in the 2D scan mode, to obtain a 2D slice of the anatomical structure;
extract features from the 2D slice; and
generate a representation of an enhanced 3D volume, wherein the enhanced 3D volume combines a 3D volume acquisition and the features extracted from the 2D slice.

The processor may be configured to switch the 3D ultrasound probe to the 2D scan mode and control the 3D ultrasound probe, in the 2D scan mode, to obtain a 2D slice of the anatomical structure within 0.2 seconds from obtaining a 3D volume acquisition.

The processor may be further configured to control the 3D ultrasound probe, in the 2D scan mode, to obtain a second 2D slice at a different plane from the 2D slice based on the extracted features.

The processor may be further configured to extract volume features from a 3D volume acquisition. The processor may also be configured to switch the 3D ultrasound probe from the 3D scan mode to the 2D scan mode based on the extracted volume features.

The processor may be further configured to switch the 3D ultrasound probe to a second 3D scan mode, after obtaining the 2D slice, and control the 3D ultrasound probe, in the second 3D scan mode, to obtain a second 3D volume acquisition of the anatomical structure, wherein the second 3D scan mode has a higher beam density near the position of the plane of the 2D slice and a lower beam density away from the position of the plane of the 2D slice.

The system may also comprise the 3D ultrasound probe. The system may also comprise a display for displaying the 2D slice and/or the enhanced 3D volume.

Herein is also provided a method for multiplanar reformation, MPR, slicing, the method comprising controlling a 3D ultrasound probe, in a first 3D scan mode, to obtain a first 3D volume of an anatomical structure and determining a target plane relative to anatomical structure (e.g., from features extracted from the first 3D volume), The method further comprises controlling the 3D ultrasound probe, in a second 3D scan mode, to obtain a second 3D volume of an anatomical structure, wherein the second 3D scan mode is configured to provide a higher beam density at, or at least near to, the target plane relative to the beam density used in the first 3D scan mode. The method further comprises MPR slicing the second 3D volume at, or at least near to, the target plane to obtain a 2D slice of the target volume at, or at least near to, the target plane. Optionally, the 2D slice may be displayed. Optionally, an indication of the position of the 2D slice, in relation to the first and/or second 3D volume, may also be displayed together with the corresponding first and/or second 3D volume.

The method for MPR slicing may be a computer-implemented method.

Herein is also provided a system for MPR slicing, the system comprising a processor configured to perform all of the steps of the method for MPR slicing. The system may also comprise the 3D ultrasound probe. The system may also comprise a display for displaying the 2D slice.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a method for enhancing ultrasound information from an anatomical structure;
Fig. 2 shows a 3D probe acquiring a 2D slice of an anatomical structure;
Fig. 3 shows 2D slices taken at different orientations with respect to the 3D volume;
Fig. 4 shows a display of the 3D volume together with two 2D slices and the indications of the positions of the 2D slices in relation to the 3D volume;
Fig. 5 illustrates the imaging of a first volume and a second, smaller, volume of an anatomical structure; and
Fig. 6 shows a method for MPR slicing.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method of ultrasound imaging. The method comprises obtaining 3D volume acquisitions of an anatomical structure with a 3D ultrasound probe and, between 3D volume acquisitions, switching the 3D ultrasound probe to a 2D scan mode and controlling the 3D ultrasound probe, in the 2D scan mode, to obtain a 2D slice of the anatomical structure. The method further comprises extracting features from the 2D slice and generating a representation of an enhanced 3D volume, wherein the enhanced 3D volume combines a 3D volume acquisition and the features extracted from the 2D slice.

A 2D plane ultrasound scan has a similar amount of scan lines as used in 3D imaging. However, the settings of the probe can be adapted based on the plane and the temporal resolution is much higher than currently realistic in 3D imaging, thereby reducing motion artefacts. This allows one to see details on a 2D scan which are very difficult to visualize within 3D scan.

It has been realized that combining the advantages of both scan types (3D + 2D) leads to the best of both worlds and can provide both accurate imaging of the anatomical structure (in the 2D scan) whilst also providing an overview of the surrounding structures (in the 3D volume).

However, in order to provide both the accurate image of the anatomical structure as well as the overview of the surrounding structures at the same time, it is preferable that the 3D volume and the 2D slice are registered (i.e., their respective positions in space are known). Registering a 2D slice to a 3D volume requires additional processing resources and may not always be accurate. For example, the features present in some 2D slices may be very similar even when the corresponding plane is translated. As such, an estimate of the position of the plane with respect to a corresponding 3D volume may not be accurate in these cases.

Thus, it is proposed that a 3D ultrasound probe is controlled to switch between a 3D scan mode and a 2D scan mode such that the 2D slice (from the 2D scan mode) is obtained between 3D volume acquisitions (from the 3D scan mode). This makes it possible to assume that the 2D slice is already registered to the 3D volume as it has been obtained by the same probe and within a short period of time.

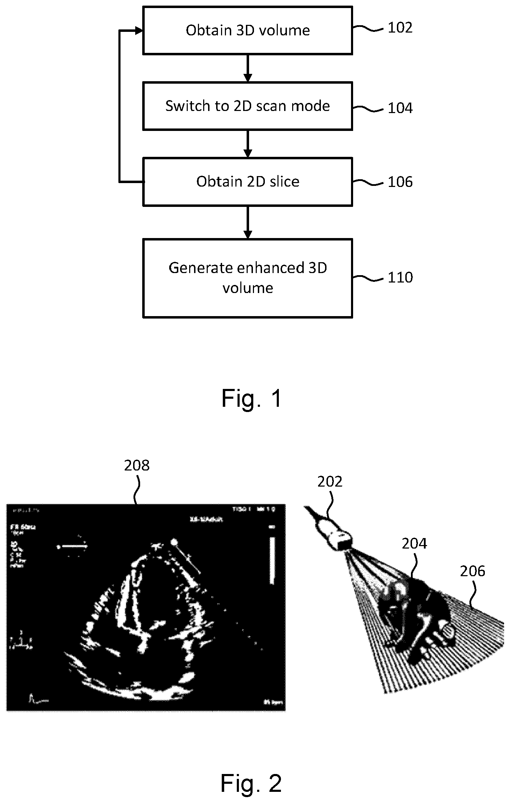

Fig. 1 shows a method for enhancing ultrasound information from an anatomical structure. A 3D volume is obtained from a 3D ultrasound probe in a 3D scan mode in step 102. The initial geometry of the model (of the anatomical structure) can then be obtained from a segmentation of a 3D volume. 3D+t ultrasound scans are the standard diagnostic tool during some procedures, so there is no additional time involved for the clinicians to obtain them. However, these scans do not show all required geometrical details for proper modelling. Other scan modalities are not an option because of practical reasons (e.g., magnetic resonance imaging, MRI, is often impractical) or of practical and x-ray exposure reasons (e.g., computed tomography, CT).

2D ultrasound provides much more geometrical detail, however it only shows a single slice. Using the 'coarse' geometry of the 3D+t scan supplemented with specific geometrical details of the 2D scan would combine the best of both worlds.

A 2D ultrasound scan with the proper settings and orientation can visualize features of the anatomical structures much better. These features are often fundamental with regard to the prediction of patient-specific outcome of clinical procedures. Without these features, the predictive value of these models will be significantly lower, up to a point that prediction cannot be made.

Thus, the 3D ultrasound probe is switched to a 2D scan mode in step 104 and a 2D slice is obtained in step 106. The 2D slice is obtained between 3D volume acquisitions (e.g., within 0.05s). It has been realized that obtaining the 2D slice does not take a significant amount of time. As such, the 2D slice can be obtained between the 3D frames without significantly affecting the acquisition/frame rate of the 3D scan mode.

This provides an automatic registering of the 2D slice to the 3D volume. The frame rate of the 3D scan mode is not significantly affected as the 2D slice can be obtained between the 3D frames and there is no need for additional processing to register the 2D slice to the 3D volume.

An enhanced 3D volume can thus be generated in step 110. This is achieved by extracting features from the 2D slice (e.g., anatomical and/or geometrical features) and combining the 3D volume with the extracted features. For example, a portion of the 3D volume may be replaced with the extracted features from the 2D slice or the extracted features may be added to the 3D volume. The 3D probe can be controlled to continuously switch between the 3D scan mode and the 2D scan mode.

The 2D slice can also be displayed together with the enhanced 3D volume. This further enables the user to visualize the anatomical structure accurately as well as providing an overview of the surrounding structures.

Software can be used to detect (i.e., extract) the features (e.g., specific geometrical detail) within the 2D slice and add that information to the 3D real-time volume.

Fig. 2 shows a 3D probe 202 acquiring a 2D slice 208 of an anatomical structure 204. The 3D probe 202 may be able to control the orientation and position of the 2D slice 208 with respect to the anatomical structure 204. For example, the angle of the plane 206 of the 2D slice can be altered (e.g., using xPlane ultrasound acquisition). Similarly, the plane 206 could be rotated around the anatomical structure 204 (e.g., using iRotate ultrasound acquisition).

A 3D scan has temporal resolution of approximately 15 frames per heartbeat. For a 2D scan, this is much higher. As such, adding 2D scans in between the 3D scans only leads to a small reduction in temporal resolution of the 3D scan. Hence, it hardly influences the real time 3D volume representation. The 2D scan can have settings which optimize the visibility of certain specific structures. These specific structures can then be visualized in the 3D volume scan by, for example, adding a spherical colored marker at the specific location. As the 2D scan time is very short, the data can be added to the 3D volume representation of the 3D image which was taken immediately before/after the 2D image.

As the same ultrasound sensor is used for the 3D and 2D scan, the orientation and position of the 2D scan is always known with respect to the 3D scan.

Thus, the idea is to (continuously) switch the scan mode between a 3D scan and a 2D scan. Software can then extract the specific geometrical detail out of the 2D scan and add it to the 3D scan.

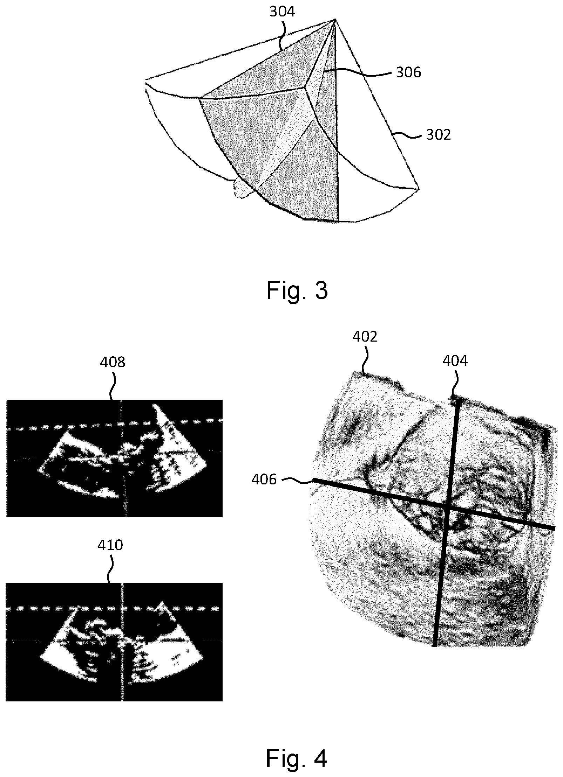

Fig. 3 shows 2D slices 304 and 306 taken at different orientations with respect to the 3D volume 302. Both 2D slices 304 and 306 could be acquired between two sequential 3D acquisitions.

Fig. 4 shows a display of the enhanced 3D volume 402 together with two 2D slices 408 and 410 and indications (404 and 406) of the positions of the 2D slices in relation to the enhanced 3D volume 402. Thus, the details of the features in the 2D slices 408 and 410 can be further visualized together with an overview of the surrounding structures using the enhanced 3D volume.

In an example, the 2D slices 408 and 410 can show geometric details of a heart valve whilst the indications 404 and 406 can shows the positions of the 2D slices in relation to the enhanced 3D volume. The enhanced 3D volume could also include the geometric details of the heart valve (and/or other geometric/anatomical features).

Switching between the 3D scan mode and 2D scan mode can be also adapted such that, instead of switching using the sequence [3D, 2D, 3D, 2D, ...] one could switch using the sequence [3D, 3D, 3D, 2D, ...] or the sequence [3D, 2D, 2D, 3D, 2D, ...]. Of course, other sequences can be used.

In an embodiment, all, or part of, geometric information of the 2D scan can be included in the 3D visualization (e.g., by replacing part of 3D data with the improved resolution 2D slice).

The method is not limited to imaging a particular anatomical structure. For example, the method can be used for cardiac ultrasound imaging, fetal ultrasound imaging etc.

In an embodiment, the 3D volume can be searched to find a target plane relative to the 3D volume. A 2D slice can then be obtained at the target plane. Machine learning could be applied to find the most suitable 2D scan planes in the 3D volume.

When searching for a particular feature of the anatomical structure, one could focus on the detail or on the area of the detail. Using a limited amount of scan lines could already be sufficient to be able to add such a detail.

Practically, for direct use during a clinical echographic investigation, one could create a slightly transparent representation of the 3D volume, enhanced by one or more typically colored plane(s) which are not, or less, transparent. Specific features could maybe also be detected by AI and added in the live 3D volume as a marker.

For indirect use (e.g., after the clinical echographic investigation), the additional 2D information can be used to enhance the 3D volume and to add locations of features which are not well visible in the normal 3D scan.

The 3D volume may be a segmentation of the 3D acquisition. For example, the anatomical structure (e.g., a heart) can be segmented from the 3D frames and the 3D model of the anatomical structure can be treated as the 3D volume.

Instead of a 2D slice, a zoomed in 3D volume could be obtained.

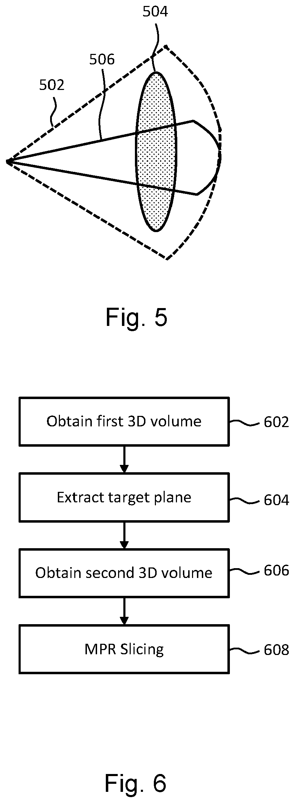

Fig. 5 illustrates the imaging of a first volume 502 and a second, smaller, volume 506 of an anatomical structure 504. The second, smaller, volume 506 is obtained with a higher beam density. The position of the second volume 506, in relation to the anatomical structure 504, may be based on features extracted from the first volume 502. For example, the second volume may be directed to areas of interest of the anatomical structure 504. Multi-planar reformation (MPR) can then be used to obtain a 2D slice from the second volume 506. The second volume 506 may be obtained with an inhomogeneous beam density, where the beam density is higher at, or near to, a target plane. The target plane can be determined from volume features extracted from the first volume 502.

Herein is also provided a method for MPR slicing.

In an example, when a transducer is placed to allow apical acquisitions, clinicians are trained to rotate transducer 60 degrees to the left and 60 degrees to the right to acquire standard apical views, A4C, A3C, etc. and derive thereof the necessary diagnostic information. This information means that clinicians are much more likely to be interested in certain areas of the anatomical structure than others. Thus, rather than improving the resolution of all possible areas of the anatomical structure, it is possible to improve the resolution of the areas clinicians are interested at the cost of other areas likely to be of lower interest to the clinicians.

Sampling 3D volumes takes time and parallel sampling has limitations and is, to a good extent, already being heavily employed. Thus, information of which areas of the anatomical structure is important can be used to automate complete cardiac acquisition. Features of the anatomical structure can be extracted from the first volume and the position/orientation of the second volume can be chosen based on the features (e.g., to include important features/planes).

Fig. 6 shows a method for MPR slicing. A first 3D acquisition is obtained in step 602. The first 3D volume may be a homogenous (anatomically ignorant) sampling that provides a full volume of the anatomical structure with low image quality. A target plane can then be extracted from the first volume in step 604 (e.g., from features in the first 3D volume). The relative position between features in the first volume can be used to extract the target plane. A confidence check can be run to ensure the target plane has been correctly extracted.

A second 3D acquisition is triggered in step 606. The second 3D acquisition may be with inhomogeneous (anatomically intelligent) sampling which grants certain areas of the anatomical structure with a higher transmit beam density (diverging beams) than others. For example, views which are around standard apical planes will get a higher number of transmit beams (and, therefore, better image quality) while other areas will have a reduced density (and lower image quality). Subsequent MPR slicing in step 608 (onto standard planes) will automatically, without further processing, provide higher image quality for those planes.

Areas that are outside of the heart (e.g., as identified by a heart model) could stop being scanned to save beams for the important area(s).

The transmit scheme could be adaptative both in space and time. Thus, in addition to improving line density, important slices could be granted a faster pulse repetition interval (PRI), for example, to better sample a fast-moving object (e.g., a valve) that is poorly sampled with a standard 3D scheme. In other words, the second 3D acquisition may have both a higher line density as well as a higher pulse repetition interval.

The second 3D acquisition may have an inhomogeneous line density, where the line density is higher for areas of interest of the anatomical structure. The areas of interest may be detected in the first 3D acquisition. The skilled person would be readily capable of developing a processor for carrying out any herein described method. Thus, each step of a flow chart may represent a different action performed by a processor and may be performed by a respective module of the processing processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method of ultrasound imaging, the method comprising:
controlling a 3D ultrasound probe, in a 3D scan mode, to obtain (102) 3D volume acquisitions of an anatomical structure;
switching (104) the 3D ultrasound probe to a 2D scan mode between 3D volume acquisitions and controlling the 3D ultrasound probe, in the 2D scan mode, to obtain (106) a 2D slice of the anatomical structure;
extracting features from the 2D slice; and
generating (110) a representation of an enhanced 3D volume, wherein the enhanced 3D volume combines a 3D volume acquisition and the features extracted from the 2D slice.

2. The method of claim 1, comprising switching the 3D ultrasound probe to the 2D scan mode and controlling the 3D ultrasound probe, in the 2D scan mode, to obtain a 2D slice of the anatomical structure within 0.2 seconds from obtaining a 3D volume acquisition.

3. The method of claims 1 or 2, further comprising controlling the 3D ultrasound probe, in the 2D scan mode, to obtain a second 2D slice at a different plane from the 2D slice based on the extracted features.

4. The method of any of claims 1 to 3, further comprising switching the 3D ultrasound probe from the 2D scan mode to the 3D scan mode based on the extracted features.

5. The method of any of claims 1 to 4, further comprising extracting volume features from a 3D volume acquisition, wherein switching the 3D ultrasound probe from the 3D scan mode to the 2D scan mode is based on the extracted volume features.

6. The method of claim 5, further comprising determining a target plane and corresponding acquisition parameters based on the extracted volume features, wherein the 3D ultrasound probe is controlled, in the 2D scan mode, to obtain a 2D slice of the anatomical structure at the target plane using the acquisition parameters.

7. The method of any of claims 1 to 6, wherein generating a representation of an enhanced 3D volume comprises adapting the 3D volume acquisition to a first transparency and superimposing the 2D slice on the 3D volume acquisition.

8. The method of any of claims 1 to 7, further comprising switching the 3D ultrasound probe to a second 3D scan mode, after obtaining the 2D slice, and controlling the 3D ultrasound probe, in the second 3D scan mode, to obtain a second 3D volume acquisition of the anatomical structure, wherein the second 3D scan mode has a higher beam density near the position of the plane of the 2D slice and a lower beam density away from the position of the plane of the 2D slice.

9. The method of claim 8, wherein the second 3D scan mode has a higher pulse repetition interval than the 3D scan mode.

10. A computer program carrier comprising computer program code means which, when executed on a processing system, cause the processing system to perform all of the steps of the method according to any of the preceding claims.

11. A system for ultrasound imaging, the system comprising a processor configured to:
control a 3D ultrasound probe, in a 3D scan mode, to obtain (102) 3D volume acquisitions of an anatomical structure;
switch (104) the 3D ultrasound probe to a 2D scan mode between 3D volume acquisitions and control the 3D ultrasound probe, in the 2D scan mode, to obtain (106) a 2D slice of the anatomical structure;
extract features from the 2D slice; and
generate (110) a representation of an enhanced 3D volume, wherein the enhanced 3D volume combines a 3D volume acquisition and the features extracted from the 2D slice.

12. The system of claim 11, wherein the processor is configured to switch the 3D ultrasound probe to the 2D scan mode and control the 3D ultrasound probe, in the 2D scan mode, to obtain a 2D slice of the anatomical structure within 0.2 seconds from obtaining a 3D volume acquisition.

13. The system of claims 11 or 12, wherein the processor is further configured to control the 3D ultrasound probe, in the 2D scan mode, to obtain a second 2D slice at a different plane from the 2D slice based on the extracted features.

14. The system of any of claims 11 to 13, wherein the processor is further configured to extract volume features from a 3D volume acquisition and wherein the processor is configured to switch the 3D ultrasound probe from the 3D scan mode to the 2D scan mode based on the extracted volume features.

15. The system of any of claims 11 to 14, wherein the processor is further configured to switch the 3D ultrasound probe to a second 3D scan mode, after obtaining the 2D slice, and control the 3D ultrasound probe, in the second 3D scan mode, to obtain a second 3D volume acquisition of the anatomical structure, wherein the second 3D scan mode has a higher beam density near the position of the plane of the 2D slice and a lower beam density away from the position of the plane of the 2D slice.
